# EUROPÄISCHE PATENTSCHRIFT

(11) **EP 4 000 673 B1**
(45) Veröffentlichungstag und Bekanntmachung des Hinweises auf die Patenterteilung: **12.06.2024**
(21) Anmeldenummer: 21020555.5
(22) Anmeldetag: 08.11.2021
(51) Int. Cl.: A61M 16/00, A61M 16/06, A61M 16/20

(54) **VORRICHTUNG ZUR VORGABE EINER CPAP-BEATMUNG MIT EINEM MINDESTVOLUMEN**
DEVICE FOR SPECIFYING A CPAP RESPIRATOR HAVING A MINIMUM VOLUME
DISPOSITIF DE DÉTERMINATION D'UNE VENTILATION CPAP AYANT UN UN VOLUME MINIMAL

(30) Priorität: 24.11.2020 DE 102020007181
(43) Veröffentlichungstag der Anmeldung: 25.05.2022
(73) Patentinhaber: Löwenstein Medical Technology S.A., 2557 Luxembourg (LU)
(72) Erfinder: ADAMETZ, Benjamin, 22609 Hamburg (DE); MEHNERT, Marcel, 25569 Kremperheide (DE)
(74) Vertreter: Marx, Thomas

(56) Entgegenhaltungen:
- EP-A1- 3 061 481
- WO-A1-96/40337
- US-A1- 2015 273 166

## Beschreibung

Beatmungsgeräte werden für die Therapie respiratorischer Störungen eingesetzt, dabei können die Beatmungsgeräte in der nicht-invasiven und invasiven Beatmung, sowohl innerklinisch als auch außerklinisch, verwendet werden.

Bei einer Beatmung eines Patienten kann in der Regel ein Beatmungsgerät mit einem inspiratorischen Zweig für den inspiratorischen Atemgasfluss und optional mit einem Zweig für den exspiratorischen Atemgasfluss eingesetzt werden. Der Zweig für den exspiratorischen Atemgasfluss ermöglicht die Ausatmung/Exspiration eines Atemgases durch den Patienten, während der Zweig für den inspiratorischen Atemgasfluss den Patienten mit Atemgas versorgt.

Die Beatmungsgeräte können im Betrieb mit einem Schlauchsystem mit passiver Ausatemöffnung/Leckageschlauchsystem oder einem Schlauchsystem mit aktivem Ausatemventil/Ventilschlauchsystem verbunden sein.

Bei im Stand der Technik bekannten Beatmungsgeräten ist es möglich, zwischen einer Beatmung mit einem Leckageschlauchsystem und einem Ventilschlauchsystem zu wechseln. Hierfür ist es bisher jedoch notwendig, extern oder intern des Beatmungsgerätes einen Umbau/Einbau einer oder mehrerer Komponenten zum Beispiel eines Rückschlagventils vorzunehmen. Ein Leckageschlauchsystem ist dabei ein Einschlauchsystem mit definierter Leckageöffnung, durch die während der Beatmung fortlaufend Atemgas entweichen kann, um Kohlendioxid auszuspülen. Ein Ventilschlauchsystem ist ein Einschlauchsystem mit einem Schaltventil für die Exspiration oder ein Doppelschlauchsystem, bei dem ausgeatmetes Atemgas wieder dem Beatmungsgerät zur Überwachung zugeführt wird. Beatmungsgeräte weisen daher zumindest zwei Anschlussstutzen auf für entweder das Doppelschlauchsystem oder das Einschlauchsystem. Zusätzlich weisen Beatmungsgeräte Druckstutzen auf, die für die Verwendung von Einschlauchsystemen mit Ventil notwendig sind, um einen Steuerdruck auf das Ventil zu führen. Bei im Stand der Technik bekannten Beatmungsgeräten muss daher immer ein passender Adapter für das ausgewählte Schlauchsystem verwendet werden und zudem müssen ggfs. Druckstutzen verschlossen oder geöffnet werden. Bevor das Patientenschlauchsystem verbunden werden kann, muss der passende Schlauchsystemadapter installiert werden. Der Einbau/Umbau ist jedoch zeitaufwendig, fehleranfällig und bedeutet ein Anwendungshindernis. Ein weiterer Nachteil ist, dass der Therapie-Modus immer abhängig vom Schlauchsystem ist. So benötigt ein CPAP-Modus immer ein Leckageschlauchsystem, damit fortlaufend Atemgas entweichen kann, um Kohlendioxid auszuspülen. Ein CPAP-Modus ist derzeit nicht mit einem Einschlauchsystem mit einem Schaltventil möglich. Beim CPAP-Modus ist eine Spontanatmung durch den Patienten zwingend notwendig für den Gasaustausch. Aussetzer oder eine Reduktion der Spontanatmung führen zu reduziertem Gasaustausch und einem Risiko für den Patienten. WO 9640337 A1 offenbart ein System für eine Beatmung mit zwei unterschiedlichen Druckniveaus und einer Überwachung des Minutenvolmens, um bei Abweichungen von einem Sollwert den IPAP anzupassen.

Aufgabe der Erfindung ist es daher, ein Beatmungsgerät bereitzustellen, welches es ermöglicht, eine abnehmende Spontanatmung im CPAP-Modus zu erkennen und eine medizinisch notwendige Mindestventilation für den Patienten, auch bei abnehmender Spontanatmung, aufrecht zu erhalten.

Aufgabe der Erfindung ist es daher, eine Vorrichtung bereitzustellen, die es ermöglicht, ein Beatmungsgerät sowohl mit einem Leckageschlauchsystem als auch mit einem Ventilschlauchsystem ohne Umbaumaßnahmen oder ohne Adapter zu nutzen und zudem eine CPAP-Therapie mit demselben Schlauchsystem zu ermöglichen.

Diese Aufgabe wird durch ein Beatmungsgerät gemäß dem Anspruch 1 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Beatmungsgerät zur Atemgasversorgung ist im unabhängigen Anspruch 1 definiert und umfasst eine Atemgasquelle, eine Steuereinheit, einen Speicher, eine Drucksensoreinrichtung und/oder eine Flusssensoreinrichtung, einen auswechselbaren Atemgasschlauch, zumindest einen Anschlussstutzen, für den Atemgasschlauch und ein Patienteninterface, wobei die Steuereinheit die Atemgasquelle zur Vorgabe eines im Wesentlichen konstanten CPAP-Drucks, der unabhängig von der Atemphase des Patienten aufrechterhalten wird, ansteuert und wobei die Steuereinheit eingerichtet und ausgebildet ist, aus den Signalen der Drucksensoreinrichtung und/oder der Flusssensoreinrichtung
- die Atemphase - Inspiration und Exspiration - des Patienten zu ermitteln,
- das aktuelle Atemzugvolumen des Patienten während aufeinanderfolgender Inspirationen und Exspirationen zu ermitteln
- zumindest eine erste, vorgegebene Volumenschwelle für das Atemzugvolumen mit dem aktuellen Atemzugvolumen zu vergleichen
- zu bestimmen, ob das aktuelle Atemzugvolumen die erste Volumenschwelle unterschreitet,
- in Reaktion auf eine Unterschreitung,
- die Atemgasquelle zur Vorgabe eines zweiten Drucks (IPAP) für das Atemgas für die Inspiration anzusteuern
- die Atemgasquelle zur Vorgabe des CPAP-Drucks für das Atemgas für die Exspiration anzusteuern.

Das Beatmungsgerät ist so eingerichtet, dass die Steuereinheit eingerichtet und ausgebildet ist den zweiten Druck (IPAP) schrittweise so lange zu erhöhen, bis die vorgegebene Volumenschwelle für das Atemzugvolumen erreicht ist.

Optional oder ergänzend ist das Beatmungsgerät auch eingerichtet, dass die Steuereinheit den zweiten Druck (IPAP) von einer Inspiration zur unmittelbar nachfolgenden Inspiration erhöht.

Das Beatmungsgerät kann auch so eingerichtet sein, dass die Steuereinheit den zweiten Druck (IPAP) schrittweise wieder absenkt, wenn die vorgegebene Volumenschwelle für das Atemzugvolumen überschritten ist.

Erfindungsgemäß ist auch vorgesehen, dass die Steuereinheit den zweiten Druck (IPAP) auf das CPAP Druckniveau absenkt, wenn die vorgegebene Volumenschwelle für das Atemzugvolumen in einer vorgegeben Weise überschritten ist und insofern wieder die Atemgasquelle zur Vorgabe eines im Wesentlichen konstanten CPAP-Drucks, der unabhängig von der Atemphase des Patienten aufrechterhalten wird, ansteuert.

Erfindungsgemäß ist vorgesehen, dass das Beatmungsgerät zumindest ein Ventil aufweist, welches in dem Atemgasschlauch oder in dem Beatmungsgerät angeordnet ist.

Erfindungsgemäß ist das Beatmungsgerät so eingerichtet, dass der Atemgasschlauch bei dem Wechsel von einem Modus CPAP zu einem IPAP-Modus an dem Beatmungsgerät verbleibt und das Patienten-Ventil für den IPAP-Modus von der Steuereinheit geschaltet wird.

Das Beatmungsgerät kann auch so eingerichtet sein, dass das Ventil abhängig von der Atemphase geöffnet oder geschlossen wird.

Das Beatmungsgerät kann ergänzend auch so eingerichtet sein, dass das Ventil in der Inspiration geschlossen wird und in der Exspiration kontrolliert angesteuert und zeitweise geöffnet wird, um eine Ausatmung zu gewährleisten.

Erfindungsgemäß ist das Beatmungsgerät so eingerichtet, dass die Patientenatmung von der Steuereinheit aus dem Verlauf eines Flusssignales der Flusssensoreinrichtung identifiziert wird und das Ventil abhängig von dem Flusssignal (als Trigger) betätigt wird.

Erfindungsgemäß ist das Beatmungsgerät ergänzend eingerichtet, dass für das Flusssignal und/oder für ein Drucksignal Grenzwerte hinterlegt sind oder einstellbar sind, wobei die Grenzwerte die Triggersensitivität sind.

Optional oder ergänzend ist das Beatmungsgerät so eingerichtet, dass die Steuereinheit zur Sicherstellung der Aufrechterhaltung des CPAP-Druckniveaus während der Schaltvorgänge des Ventils die Atemgasquelle ansteuert.

Optional oder ergänzend ist das Beatmungsgerät auch so eingerichtet, dass die Steuereinheit den CPAP-Druck zumindest zeitweise absenkt, wenn die Patientenatmung von der Steuereinheit aus dem Verlauf des Flusssignales der Flusssensoreinrichtung als Exspiration identifiziert wird.

Optional oder ergänzend ist das Beatmungsgerät so eingerichtet, dass die Steuereinheit den CPAP-Druck zumindest zeitweise anhebt (Lippenbremse), wenn die Patientenatmung von der Steuereinheit aus dem Verlauf des Flusssignales der Flusssensoreinrichtung als Exspiration identifiziert wird.

Erfindungsgemäß ist das Beatmungsgerät so eingerichtet, dass die Steuereinheit den CPAP-Druck auf Druckwerte unter 4 hPa vorgeben kann, da durch das Ventil eine Auswaschung von CO der Ausatemluft auch bei geringen Drücken sicher erfolgt.

Optional oder ergänzend ist das Beatmungsgerät so eingerichtet, dass die Steuereinheit für den Modus CPAP das Ventil in der Inspiration geschlossen hält und in der Exspiration kontrolliert ansteuert und zeitweise öffnet, um eine Ausatmung zu gewährleisten, wobei die Patientenatmung, von der Steuereinheit , aus dem Verlauf des Flusssignales der Flusssensoreinrichtung identifiziert und das Ventil abhängig von dem Flusssignal (als Trigger) betätigt wird, wobei zur Sicherstellung der Aufrechterhaltung des CPAP-Druckniveaus, während der Schaltvorgänge des Ventils die Atemgasquelle angesteuert wird.

Erfindungsgemäß ist auch vorgesehen, dass eine Atemanstrengung (Inspirationsbemühung) des Patienten von der Steuereinheit aus dem Verlauf des Flusssignales oder des Drucksignales identifiziert wird und die Steuereinheit die Atemgasquelle unter Vorgabe eines Atemgasflusses oder Atemgasdrucks ansteuert, wenn aus dem Verlauf des Flusssignales oder des Drucksignales eine Inspirationsbemühung des Patienten identifiziert ist.

Die Erfindung sieht auch vor, dass der Druck der Atemunterstützung und das Volumen einstellbar sind.

Optional oder ergänzend ist das erfindungsgemäße Beatmungsgerät so eingerichtet, dass der Druck der Atemunterstützung und eine Inspirationszeit Ti einstellbar sind.

Optional oder ergänzend ist das Beatmungsgerät so eingerichtet, dass für die Exspiration das Ventil kurz geöffnet wird, sodass der Druck abgebaut ist, und das Ventil anschließend geschlossen wird.

Das Beatmungsgerät ist auch dadurch gekennzeichnet, dass die Triggersensitiviät in 3 bis 15 Stufen einstellbar ist.

Das Beatmungsgerät ist ergänzend dadurch gekennzeichnet, dass eine Triggersperrzeit (im Bereich 0,1 bis 10 Sekunden) vorgebbar ist, wobei für die Zeitdauer der Triggersperrzeit Atemanstregungen des Patienten von der Steuereinheit ignoriert werden
Optional oder ergänzend ist das Beatmungsgerät so eingerichtet, dass die Steuereinheit die Atemgasquelle zur Vorgabe eines Atemgasdruckes im Bereich von 0 - 0 mbar, bevorzugt - 0 mbar, besonders bevorzugt - 0 mbar ansteuert.

Optional oder ergänzend ist das Beatmungsgerät auch so eingerichtet, dass es eine Druckgasquelle und zumindest einen Druckschlauch aufweist, der einen Steuerdruck zu dem Ventil leitet.

Das Beatmungsgerät ist auch dadurch gekennzeichnet, dass die Atemgasquelle die Druckgasquelle ist.

Optional oder ergänzend ist das Beatmungsgerät so eingerichtet, dass der Atemgasschlauch ein Einschlauchsystem mit Ventil ist.

Optional oder ergänzend ist das Beatmungsgerät so eingerichtet, dass der Atemgasschlauch ein Zweischlauchsystem mit Ventil ist.

Erfindungsgemäß ist das Beatmungsgerät so eingerichtet, dass der Atemgasschlauch ein Zweischlauchsystem mit einem zugeordneten Ventil ist, wobei sich das Ventil in einem Beatmungsgerätegehäuse benachbart zu dem Stutzen befindet.

Optional oder ergänzend ist das Beatmungsgerät so eingerichtet, dass das Patienten-Ventil aus einer Aufnahme des Gehäuses entnehmbar ausgebildet ist, wobei das Patienten-Ventil eine Membran aufweist, die mit einem Steuerdruck beaufschlagt werden kann, um einen Atemgasfluss über das Ventil zu sperren oder freizugeben.

Erfindungsgemäß ist das Beatmungsgerät so eingerichtet, dass das Ventil eine Dichtmembran aufweist, die mit einem Steuerdruck beaufschlagt wird, der das Ventil öffnet oder schließt, wobei der Steuerdruck von der Atemgasquelle erzeugt wird und über einen nicht Steuerschlauch zum Ventil geleitet wird.

Das Beatmungsgerät kann auch ein Ventil aufweisen, welches elektrisch betrieben wird.

Erfindungsgemäß ist das Beatmungsgerät so eingerichtet, dass das Patienteninterface als Nasenkanüle oder Flusskanüle, als Nasenstopfen oder Maske oder als Tracheostomieanschluss ausgebildet ist.

In einer weiteren Weiterbildung weist das Beatmungsgerät eine Benutzerschnittstelle auf, die als Bedien-/und Anzeigeelement eingerichtet und ausgebildet ist. In der Regel ist das Bedien-/ und Anzeigeelement als Graphical User Interface (GUI) ausgebildet. In der Regel ist das GUI als Touchscreen ausgebildet. Optional umfasst das Bedien-/ und Anzeigeelement haptische Bedienelemente.

In Ausgestaltung umfasst das Beatmungsgerät eine digitale Schnittstelle, die eingerichtet ist, erfasste Parameter, Messwerte und Informationen an einen Server oder ein externes Endgerät zu übermitteln und Daten und Informationen über die Schnittstelle zu empfangen. Optional ist das Beatmungsgerät eingerichtet, die erfassten Werte und/oder Informationen der Messstrecke zu speichern, zu analysieren und/oder zu bewerten. Das Beatmungsgerät kann über die Schnittstelle mit einem Hustengerät oder einem anderen Beatmungsgerät oder einem Patientenmonitor gekoppelt werden und Daten austauschen. Optional ist das Beatmungsgerät eingerichtet, die erfassten, analysierten und/oder bewerteten Messwerte/Parameter an einen externen Server zu übermitteln. Die Übermittlung kann dabei zeitgesteuert, manuell ausgelöst (z.B. am Heimtherapiegeräte oder am Server ausgelöst), ereignisgesteuert (z.B. bei Erkennung bestimmter kritischer Zustände durch das Therapiegerät) oder als dauerhafte Übertragung, zumindest während einer laufenden Therapie, eingerichtet sein.

Eine Übertragung der Messwerte, Parameter und Informationen kann alle 2 Stunden bis 7 Tage, insbesondere alle 1 bis 3 Tage erfolgen. In einer Ausführung erfolgt die Übertragung zumindest einmal pro Tag/pro 24 Stunden. Optional kann die Schnittstelle eingerichtet sein, Messwerte, Informationen oder Parameter stundenweise zusammenzugefasst zu übertragen oder die Messwerte in Echtzeit zu übertragen. Optional ist durch den Benutzer und/oder durch einen Betreuer ein Übertragungszyklus frei wählbar. Die Schnittstelle des Beatmungsgerätes kann eingerichtet sein, die Übertragung selbsttätig, gegebenenfalls wiederholt oder dauerhaft, nach einer oder mehreren fest einprogrammierten und/oder frei eingegebenen Zeitintervallen durchzuführen.

Im Falle eines Ausfalls einer Datenverbindung kann die Speichereinheit der Vorrichtung eingerichtet sein, die Messwerte und/oder die Informationen zumindest einen Tag zu speichern, wobei die Schnittstelle der Vorrichtung eingerichtet ist, die Messwerte an einen externen Server oder ein Endgerät zu übertragen, sobald eine Datenverbindung erneut aufgebaut wurde.

Optional ist das Beatmungsgerät eingerichtet, über das Bedien- und/ Anzeigeelement Informationen und/oder Werte, die durch den Benutzer und/oder durch den Betreuer manuell eingegeben werden, mit in die Auswertung von Messwerten einzubeziehen.

In weiterer Ausgestaltung umfasst das Beatmungsgerät eine Alarmeinheit mit einem Lautsprecher, die eingerichtet ist, bei erkannten Vorkommnissen einen Alarm auszugeben, wobei das Beatmungsgerät zumindest ein Mikrophon umfasst, dass eingerichtet ist, einen von der Alarmeinheit ausgegebenen Alarm zu überwachen. Dies bietet eine zusätzliche Sicherheitsfunktion für die korrekte Verwendung der Vorrichtung zur Beatmung.

In Ausgestaltung ist das Beatmungsgerät eingerichtet, mit weiteren Vorrichtungen kombinierbar zu sein. Optional weist das Beatmungsgerät einen Anschluss für einen Vernebler auf, wobei das Beatmungsgerät eingerichtet ist, bei einem angeschlossenen Vernebler diesen über das Beatmungsgerät zu steuern. Das Beatmungsgerät ist optional eingerichtet, eine Rückmeldung des Verneblers zu erfassen und bei der Steuerung des Verneblers zu berücksichtigen.

Das Beatmungsgerät umfasst Anschlüsse für einen Server, ein Patientenmanagementsystem, eine Hustenvorrichtung und eine Schlaflaborinfrastruktur. Zudem umfasst das Beatmungsgerät eine Cloud-Funktion, wobei das Beatmungsgerät eingerichtet ist, Daten über eine Schnittstelle an eine Cloud zu übermitteln oder einen Anschluss für ein GSM-Modul. In einer Weiterbildung umfasst das Beatmungsgerät einen Anschluss für ein Krankenschwesterruf-Modul. Zudem umfasst das Beatmungsgerät zumindest einen SpO2- und/oder einen CO2- Anschluss.

Das Beatmungsgerät weist beispielsweise folgende Betriebszustände auf:
Ein: Die Therapie läuft. Beatmungsgeräte- und Therapieeinstellungen sind möglich.
Standby: Das Gebläse ist aus und die Therapie läuft nicht. Das Beatmungsgerät ist jedoch sofort betriebsbereit. Beatmungsgeräte- und Therapieeinstellungen sind möglich.
Aus: Das Beatmungsgerät ist ausgeschaltet. Es sind keine Einstellungen möglich und das Display bleibt dunkel.

Das Beatmungsgerät ist für eine durchgehende oder intermittierende Atemunterstützung für die Versorgung von Personen vorgesehen, die mechanisch beatmet werden müssen. Das Beatmungsgerät ist beispielsweise auch für Kinder und Erwachsene mit einem minimalen Tidalvolumen von 30 ml vorgesehen. Das Beatmungsgerät eignet sich für die Anwendung im häuslichen Bereich, in Pflegeeinrichtungen und in Krankenhäusern sowie für mobile Anwendungen, beispielsweise im Rollstuhl oder auf einer Transportliege. Es kann für die invasive und nicht-invasive Beatmung zum Einsatz kommen. Das Beatmungsgerät ist auch für die Verwendung als Beatmungsgerät während des Transports oder in der Intensivpflege vorgesehen.

Das Beatmungsgerät kann sowohl mit nicht-invasiven als auch mit invasiven Beatmungszugängen verwendet werden. Ein Gebläse saugt Umgebungsluft über einen Filter an und befördert sie mit dem Therapiedruck über dem Beatmungsschlauch und den Beatmungszugang zum Patienten. Auf Basis der erfassten Signale der Druck- und Flow-Sensoren wird das Gebläse entsprechend der Atemphasen gesteuert. Die Bedienoberfläche dient zur Anzeige und Einstellung der zur Verfügung stehenden Parameter und Alarme. Das Beatmungsgerät kann sowohl mit einem Leckageschlauch, einem Einschlauch-Ventilsystem oder einem Doppelschlauchsystem verwendet werden. Beim Leckageschlauch wird über ein Ausatemsystem die CO2-haltige Ausatemluft kontinuierlich ausgespült. Beim Einschlauch-Ventilsystem und beim Doppelschlauchsystem wird die Ausatmung des Patienten über ein Ventil gesteuert.

Mit einem integrierten FiO2-Sensor lässt sich bei Bedarf die vom Beatmungsgerät abgegebene FiO2-Konzentration messen. Auch die Einspeisung einer externen SpO2-Messung ist möglich. Die Netzversorgung findet über ein externes Netzteil statt. Das Beatmungsgerät verfügt über eine eingebaute Batterie und kann daher bei Netzausfall unterbrechungsfrei weiter betrieben werden. Zusätzlich können maximal zwei externe Batterien angeschlossen werden, um das Beatmungsgerät zu betreiben. Die Therapiedaten werden im Beatmungsgerät gespeichert und können zusätzlich auf einem USB-C-Stick geladen werden und mittels PC-Software ausgewertet werden.

Der Atemgasantrieb kann ein Gebläse, ein Ventil, eine Sauerstoff-Quelle (Hochdruck) oder eine Luftdruckquelle (Hochdruck) oder eine Kombination aus den Vorgenannten sein. Der Atemgasantrieb ist beispielsweise über zumindest zwei, insbesondere drei Aufhängepunkte frei schwingend in dem Beatmungsgerät angeordnet.

Die Steuereinheit umfasst in der Regel zumindest eine Speichereinheit und eine Auswerteeinheit. Die Speichereinheit ist eingerichtet, Messwerte, Informationen und/oder Parameter zu speichern und für Auswertungen durch die Auswerteeinheit bereitzustellen. Die Auswerteeinheit ist eingerichtet, die Messwerte, Informationen und/oder Parameter miteinander oder mit externen Daten zu vergleichen. Die Steuereinheit ist eingerichtet, Daten von Komponenten der Vorrichtung, insbesondere einer Messeinheit der Flussmessstrecke zu empfangen, zu speichern und zu analysieren. Optional ist die Steuereinheit eingerichtet, Daten Messwerte, Informationen und/oder Parameter an eine digitale Schnittstelle der Vorrichtung zu übermitteln.

Das Beatmungsgerät ist insbesondere auch für die Verwendung in der pädiatrischen Beatmung eingerichtet. Die Vorrichtung umfasst hinterlegte Beatmungsmodi. Insbesondere umfasst das Beatmungsgerät zumindest einen High-Flow-Modus und zumindest einen PEEP-Steuerungsmodus. In der Regel ist die Steuereinheit der Vorrichtung eingerichtet, die Beatmungsmodi, Frequenzen, Trigger und Flüsse der Vorrichtung einzustellen.

Das Beatmungsgerät kann mit Leckageschlauch, einem Einschlauch-Ventilsystem oder einem Doppelschlauchsystem verwendet werden. Beim Leckageschlauch wird über ein Ausatemsystem die CO2-haltige Ausatemluft kontinuierlich ausgespült.

Beim Einschlauch-Ventilsystem und beim Doppelschlauchsystem wird die Ausatmung des Patienten über ein Ventil gesteuert.

Beim Doppelschlauchsystem ist das Ventil im Beatmungsgerät angeordnet. Die Ausatemluft wird über einen Teilschlauch zum Exspirations-Eingangsstutzen des Beatmungsgerätes geleitet und von dort über das Ventil in die Umgebung abgegeben. Das Ventil öffnet dafür mit jeder Exspiration. Das Ventil wird mit jeder Inspiration geschlossen. Beim Einschlauch-Ventilsystem ist das Ventil im oder am Schlauch angeordnet. Das Ventil wird, egal ob intern oder extern, immer mit einem Steuerdruck beaufschlagt, der das Ventil öffnet oder schließt.

Der Steuerdruck wird von der Atemgasquelle erzeugt und über einen Steuerschlauch zum Ventil geleitet. Dabei wird der Steuerdruck zuerst zum internen Ventil geleitet. Von dort kann der Druck zum zweiten Ventil (im Einschlauch) geleitet werden. Ein Unterbrecher gibt den Weg frei oder versperrt ihn.

Für das Einschlauchsystem ist am Beatmungsgerätegehäuse ein Druckstutzten angeordnet an dem der Steuerdruck ansteht. Über einen Druckschlauch wird der Steuerdruck zum Ventil geleitet.

Die voranstehend beschriebene Erfindung weist insgesamt den Vorteil auf, dass eine Beatmung eines Patienten ermöglicht wird, wobei eine Mobilität des Patienten aufrechterhalten werden kann. Beispielsweise kann die Vorrichtung an einem Rollstuhl angebracht sein. Die Vorrichtung umfasst beispielsweise zudem eine Absaugungsfunktion und einen Hustenmodus. Das Beatmungsgerät kann an verschiedene Schlauchsysteme angepasst werden, ohne dass ein Umbau des Anschlussbereiches des Schlauchsystems am Beatmungsgerät erfolgt.

Die Inspirationszeit (Ti) kann für die Spontanatmung eingestellt werden. Im Bereich 0,2 Sekunden bis 4 Sekunden bei Kindern und 0,5 Sekunden bis 5 Sekunden bei Erwachsenen. Die Inspiration wird spätestens nach Ablauf von Ti beendet.

Mandatorischer Atemzug: Ti ist fest eingestellt.

Die Triggersensitivität kann in 10 Stufen eingestellt werden. Ebenso kann eine Triggersperrzeit eingestellt werden. Inspiratorische Triggersignale werden im eingestellten Zeitraum, der im Bereich 0,2 s bis 5 s liegt, ignoriert.

Für die Volumenvorgabe gilt: Der inspiratorische positive Atemwegsdruck (IPAP) kann eingestellt werden im Bereich 4 - 50 hPa / mbar / cmH2O bei Verwendung des Leckageschlauchsystems oder im Bereich 4 - 60 hPa / mbar / cmH2O bei Verwendung des Ein- oder Doppelschlauch-Ventilsystem.

Das abgegebene Volumen (Vt) kann eingestellt werden. Im Bereich 30 ml bis 400 ml bei Kindern und 100 ml bis 3000 ml bei Erwachsenen.

Die Triggersensitivität kann in 10 Stufen eingestellt werden. Ebenso kann eine Triggersperrzeit eingestellt werden. Inspiratorische Triggersignale werden im eingestellten Zeitraum, der im Bereich 0,2 s bis 5 s liegt, ignoriert.

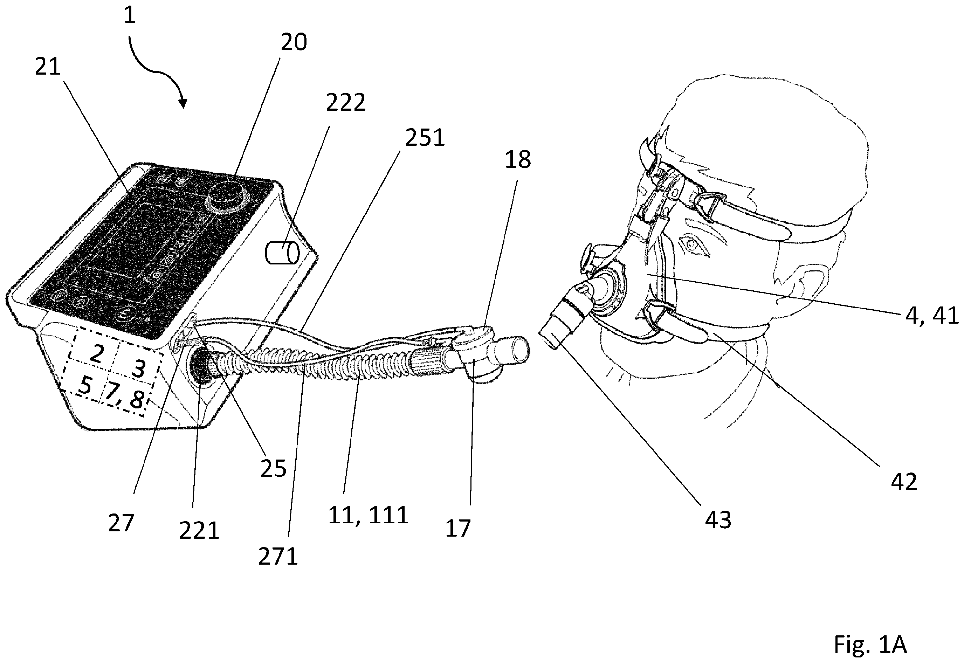

Figur 1A zeigt das erfindungsgemäße Beatmungsgerät 1 mit einer Beatmungsmaske 41 als Patienteninterface 4. Die Maske ist mit einer Bänderung 42 am Kopf befestigt. Über einen Stutzen 43 kann die Maske mit dem Schlauch verbunden werden.

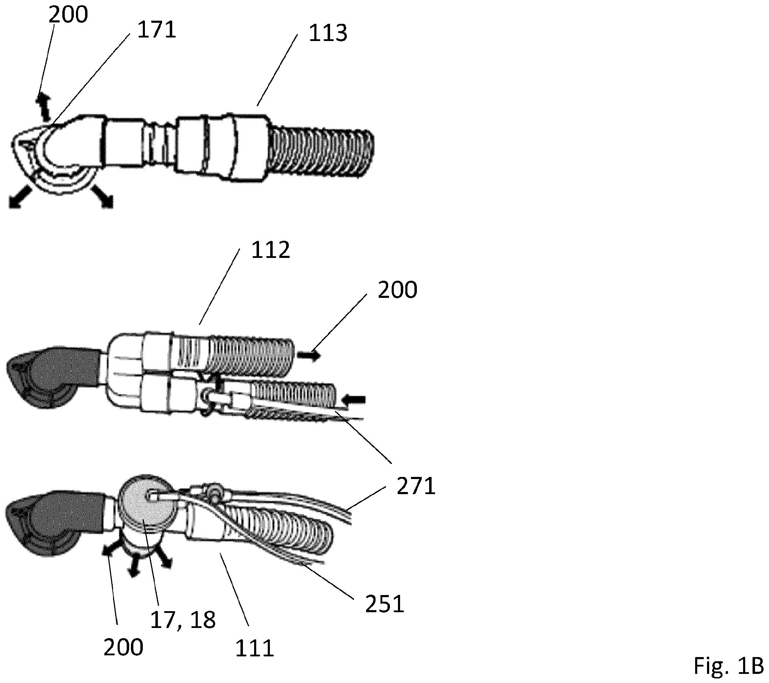

Das Beatmungsgerät zur Atemgasversorgung 1 umfasst eine Atemgasquelle 2, eine Steuereinheit 3, einen Speicher 5, eine Drucksensoreinrichtung 7 und/oder eine Flusssensoreinrichtung 8, einen Atemgasschlauch 11 und ein Patienteninterface 4, welches hier als Beatmungsmaske 41 ausgebildet ist. Das Beatmungsgerät weist zudem eine Bedieneinheit 20 und eine Anzeige 21 auf. Das Beatmungsgerät weist zudem zwei Stutzen 221, 222 für den Atemgasschlauch 11 auf. Ein Stutzen 221 ist für den Anschluss des Atemgasschlauches 11 in Form eines Einschlauch-Ventilsystems 111 eingerichtet. An diesen Stutzen 221 kann auch ein Leckageschlauch 113 (siehe Fig. 1B) angeschlossen werden.

Zudem kann an diesen Stutzen 221 der inspiratorische Zweig des Doppelschlauchsystems 112 angeschlossen werden. Der andere Stutzen 222 dient dem Anschluss des exspiratorischen Zweigs des Doppelschlauchsystems 112 (nicht gezeigt).

Das Beatmungsgerät ist eingerichtet und ausgebildet zur Atemgasversorgung 1 umfassend eine Atemgasquelle 2, eine Steuereinheit 3, einen Speicher 5, eine Drucksensoreinrichtung 7 und/oder eine Flusssensoreinrichtung 8, einen auswechselbaren Atemgasschlauch 11, zumindest einen Anschlussstutzen 221, 222 für den Atemgasschlauch und ein Patienteninterface 4, wobei die Steuereinheit 3 die Atemgasquelle 2 zur Vorgabe eines im Wesentlichen konstanten CPAP-Drucks, der unabhängig von der Atemphase des Patienten aufrechterhalten wird, ansteuert und wobei die Steuereinheit 3 eingerichtet und ausgebildet ist, aus den Signalen der Drucksensoreinrichtung 7 und/oder der Flusssensoreinrichtung 8
- die Atemphase - Inspiration und Exspiration - des Patienten zu ermitteln,
- das aktuelle Atemzugvolumen des Patienten während aufeinanderfolgender Inspirationen und Exspirationen zu ermitteln
- zumindest eine erste, vorgegebene Volumenschwelle für das Atemzugvolumen mit dem aktuellen Atemzugvolumen zu vergleichen
- zu bestimmen, ob das aktuelle Atemzugvolumen die erste Volumenschwelle unterschreitet
- in Reaktion auf eine Unterschreitung,
   ∘ die Atemgasquelle 2 zur Vorgabe eines zweiten Drucks (IPAP) für das Atemgas für die Inspiration anzusteuern
   ∘ die Atemgasquelle 2 zur Vorgabe des CPAP-Drucks für das Atemgas für die Exspiration anzusteuern.

Fig. 1B zeigt unterschiedliche Schlauchsysteme. Das Beatmungsgerät kann mit einem Leckageschlauch 113 (oben), einem Einschlauch-Ventilsystem 111 (unten) oder einem Doppelschlauchsystem 112 (Mitte) verwendet werden.

Beim Leckageschlauch 113 wird über ein Ausatemsystem 171 die CO2-haltige Ausatemluft 200 kontinuierlich ausgespült.

Beim Einschlauch-Ventilsystem und beim Doppelschlauchsystem wird die Ausatmung des Patienten über ein Ventil 17 gesteuert.

Beim Doppelschlauchsystem 112 ist das Ventil 17 im Beatmungsgerät angeordnet. Die Ausatemluft wird über einen Teilschlauch zum Exspirations-Eingangsstutzen 222 des Beatmungsgerätes geleitet und von dort über das Ventil 17 in die Umgebung abgegeben.

Das Ventil öffnet dafür mit jeder Exspiration. Das Ventil wird mit jeder Inspiration geschlossen. Ein Druckmessschlauch 271 greift den Druck im Zweischlauchsystem ab.

Beim Einschlauch-Ventilsystem 111 ist das Ventil 17 im oder am Schlauch 11 angeordnet.

Das Ventil 17 weist beispielsweise drei grundsätzliche Gaswege auf, sowie eine Öffnung, die mit einer Dichtmembran versehen ist. Die Gaswege sind ein verschließbarer exspiratorischer Gasweg, ein inspiratorischer Gasweg der zum Beatmungsgerät weist und über den inspiratorisches Atemgas strömt und ein Patienten Gasweg, der zum Patienteninterface weist. Über den Patienten Gasweg strömt in der Inspiration inspiratorisches Atemgas und in der Exspiration exspiratorisches Atemgas. Der exspiratorische Gasweg kommuniziert mit der Öffnung, die über die Membran vollständig geschlossen oder geöffnet werden kann.

Die Öffnung und die Dichtmembran sind in der Figur 1 von einer Verschlusskappe 18 verdeckt. Der Druckschlauch 251 mündet auf der Verschlusskappe 18. Der Druckschlauch 251 führt den Steuerdruck auf die Membran. Die Membran verschließt dann die Öffnung, die zum exspiratorischen Gasweg führt.

Das Ventil kann pneumatisch betrieben und/oder gesteuert werden. Das Ventil wird, egal ob im Beatmungsgerät oder am Schlauch angeordnet, beispielsweise mit einem Steuerdruck beaufschlagt, der das Ventil öffnet oder schließt. Das Ventil weist eine Dichtmembran auf, die mit einem Steuerdruck beaufschlagt wird, der das Ventil öffnet oder schließt, wobei der Steuerdruck von der Atemgasquelle 2 erzeugt wird und über einen nicht gezeigten Steuerschlauch zum Ventil 17 geleitet wird.

Ein Druckmessschlauch 271 greift den Druck im Schlauch benachbart zu dem Ventil ab. Der Steuerdruck wird von der Atemgasquelle 2 erzeugt und über einen nicht gezeigten Steuerschlauch zum Ventil geleitet. Dabei wird der Steuerdruck beispielsweise zuerst zum internen Ventil 17, welches angeordnet ist benachbart zum Exspirations-Eingangsstutzen 222, geleitet. Von dort kann der Druck auch zum zweiten Ventil 17 (im Einschlauchsystem) geleitet werden. Ein nicht gezeigter Unterbrecher gibt den Weg zum Einschlauch-Ventilsystem 111 frei oder versperrt ihn.

Für das Einschlauchsystem ist am Beatmungsgerätegehäuse ein Druckstutzen 25 angeordnet an dem der Steuerdruck ansteht. Über einen Druckschlauch 251 wird der Steuerdruck zum Ventil 17 geleitet. Der Druckstutzen 25 kann verschlossen werden, um einen Druckverlust bei Nichtgebrauch zu verhindern.

Benachbart zu dem Stutzen 221 ist auch ein Stutzen 27 für eine Druckmessung angeordnet. Im Beatmungsgerät befindet sich, dem Stutzen 27 pneumatisch zugeordnet, ein Drucksensor. An den Stutzen 27 kann ein Druckmessschlauch 271 adaptiert werden, der den Druck im Schlauch im Bereich (in Strömungsrichtung) vor oder hinter dem Ventil 17 bestimmt.

Benachbart zu dem Stutzen 222 kann auch ein Stutzen für eine Druckmessung angeordnet sein. Im Beatmungsgerät befindet sich, dem Stutzen pneumatisch zugeordnet, ein Drucksensor. An den Stutzen kann ein Druckmessschlauch 271 adaptiert werden, der den Druck im exspiratorischen Schlauch oder im Bereich (in Strömungsrichtung) vor oder hinter dem Ventil bestimmt. Diese Druckmessung ist sinnvoll, um die Einhaltung der Druckvorgabe für die Exspiration zu bestimmen und ggfs. zu regeln.

Erfindungsgemäß kann das Ventil 17 elektronisch gesteuert werden. Dann wird es beispielsweise über eine Kabelverbindung aus dem Beatmungsgerät mit Energie versorgt oder über eine Batterie, die benachbart zu dem Ventil angeordnet ist.

Alternativ kann das Ventil elektrisch betrieben und/oder gesteuert werden, beispielsweise wird die Membran dann durch einen elektrisch betriebenen Aktor gegen die Öffnung bewegt.

Alternativ kann das Ventil elektrisch, beispielsweise als axialer Voice-Coil-Aktor betrieben und/oder gesteuert werden. Diese bestehen aus einem Permanentmagneten in einer beweglichen Röhrenspule aus Draht, die sich in einem ferromagnetischen Zylinder befindet. Wenn Strom durch die Spule fließt, wird sie magnetisiert und stößt die Magneten ab. Auf diese Weise wird eine Bewegung nach innen und nach außen sowie vor und zurück erzeugt. Weitere Vorteile linearer VCA-Motoren sind ihre Bidirektionalität sowie das Vorhandensein von Permanentmagneten und magnetischen Haltespulen. Sie ermöglichen es, bei Unterbrechung der Stromversorgung an einem Ende des Hubs zu verbleiben - um zum Beispiel sicherzustellen, dass Ventile bei einem Stromausfall offen oder geschlossen bleiben. VCAs beschleunigen innerhalb des Hubs gleichmäßig und schnell, nahezu ohne Hysterese.

Das Ventil im Schlauch und/oder das Ventil im Beatmungsgerät können elektrisch gesteuert sein.

In dieser Konfiguration ist das Beatmungsgerät beispielsweise für Modus CPAP 61 eingerichtet.

Auf Anwenderauswahl hin oder automatisch aktiviert die Steuereinheit 3 den Therapie-Modus CPAP 61. Dabei wird die Atemgasquelle 2 zur Vorgabe eines konstanten CPAP-Drucks angesteuert. Bevorzugt wird der CPAP-Druck unabhängig von der Atemphase aufrechterhalten.

Bei dem Wechsel zu dem Modus CPAP 61 verbleibt der Atemgasschlauch 11 an dem Beatmungsgerät 1. Das Patienten-Ventil 17 wird dabei für die Vorgabe des Modus CPAP 61 von der Steuereinheit geschaltet.

Das Ventil 17 wird in der Inspiration geschlossen und in der Exspiration kontrolliert angesteuert und zeitweise geöffnet, um eine Ausatmung zu gewährleisten.

Dafür wird die Patientenatmung, von der Steuereinheit 3, aus dem Verlauf des Flusssignales der Flusssensoreinrichtung 8 identifiziert und das Ventil 17 wird abhängig von dem Flusssignal (als Trigger) betätigt.

Für das Flusssignal sind Grenzwerte hinterlegt oder einstellbar. Die Grenzwerte repräsentieren den Wechsel zwischen Inspiration und Exspiration und dienen somit als Triggersignale für die Ansteuerung des Ventils 17. Erfindungsgemäß ist auch ein Drucktrigger möglich oder eine Kombination beider Trigger-Optionen. Beim Drucktrigger wird die Inspiration von der Steuereinheit dadurch erkannt, dass der Druck leicht absinkt und die Exspiration wird dadurch erkannt, dass der Druck leicht ansteigt. Für das Drucksignal sind Grenzwerte hinterlegt oder einstellbar. Die Grenzwerte repräsentieren den Wechsel zwischen Inspiration und Exspiration und dienen somit als Triggersignale für die Ansteuerung des Ventils 17.

Die Steuereinheit 3 steuert die Atemgasquelle, zur Sicherstellung der Aufrechterhaltung des CPAP-Druckniveaus, während der Schaltvorgänge des Ventils 17 an.

Die Steuereinheit 3 senkt den CPAP-Druck beispielsweise zumindest zeitweise ab, wenn die Patientenatmung von der Steuereinheit 3 aus dem Verlauf des Flusssignales der Flusssensoreinrichtung 8 als Exspiration identifiziert wird. Dadurch wird die Ausatmung für den Patienten angenehmer.

Die Steuereinheit 3 hebt den CPAP-Druck alternativ beispielsweise zumindest zeitweise an (Lippenbremse), wenn die Patientenatmung von der Steuereinheit 3 aus dem Verlauf des Flusssignales der Flusssensoreinrichtung 8 als Exspiration identifiziert wird. Durch den erhöhten Druck können verschlossene Lungenbereiche eröffnet werden, die Exspiration kann dann vollständig erfolgen.

Die Steuereinheit 3 kann den CPAP-Druck auch auf Druckwerte unter 4 hPa vorgeben, da durch das Ventil 17, entsprechend dem Öffnungsgrad des Ventils, eine Auswaschung von CO2 der Ausatemluft auch bei geringen Drücken sicher erfolgt.

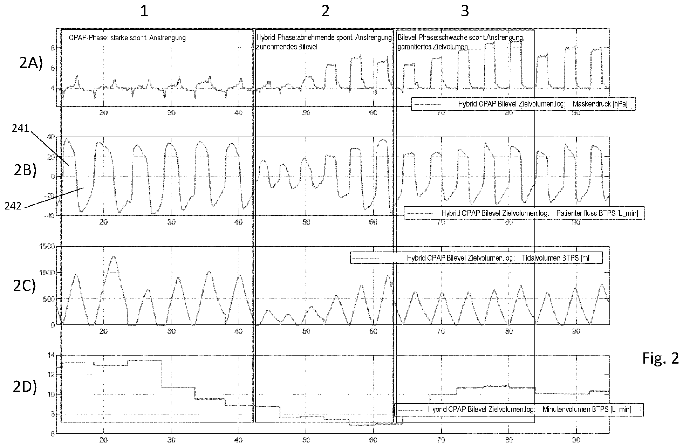

In der Figur 2 ist die Vorgabe für die CPAP-Therapie dargestellt.

In der Figur 2A ist auf der Y-Achse der Druck dargestellt. Aufgetragen ist der Maskendruck 32, welcher von dem Drucksensor 7 bestimmt wird. (Wobei der Solldruck 33, der als Vorgabewert der Steuereinheit 3 für die Atemgasquelle vorgegeben wird, dem Maskendruck zumindest phasenweise zumindest annähernd entspricht). Der Druck ist in der Einheit hPa angegeben.

In der Phase 1 liegt der CPAP-Druck (von 4hPa) an und der Patient atmet spontan. Man erkennt in Phase 1, dass der CPAP-Druck an der Maske im Wechsel von Inspiration 241 zu einer Exspiration 242 (siehe Fig. 2B) leicht schwankt. Mit jeder beginnenden Inspiration 241 sinkt der Druck unter den Solldruck ab, da der Patient mit seiner Spontanatmung einen negativen Sog entwickelt, dem der Druckregler nicht so schnell folgen kann.

Mit jeder beginnenden Exspiration 242 steigt der Druck über den Solldruck an, da der Patient mit seiner Spontanatmung einen positiven Strom (in die Maske) entwickelt, dem der Druckregler nicht so schnell folgen kann.

In der Figur 2B ist auf der Y-Achse der Fluss (in L pro Minute) dargestellt, aus dem die Atemphase 24 des Patienten erkannt werden kann. Die Atemphase 24 setzt sich zumindest aus einer Inspiration 241 und/oder einer Exspiration 242 zusammen.

Aus einem Vergleich der Figuren 2B und 2A wird ersichtlich, dass der Wechsel von einer Inspiration 241 zu einer Exspiration 242 regelmäßig im Rhythmus der Spontanatmung des Patienten stattfindet. Eine Inspiration 241 entspricht positiven Flüssen. Eine Exspiration 242 entspricht negativen Flüssen.

In der Figur 2C ist auf der Y-Achse das Volumen in ml aufgetragen. Man erkennt, dass das Volumen in der Phase 1 in der Inspiration 241 immer über 500 ml ist. In der Phase 2 sinkt das Volumen unter 500 ml. Ein Volumen von 500 ml ist hier beispielhaft als Grenzwert eingestellt. Da 500 ml als Grenzwert eingestellt ist, unter den das Volumen bei CPAP-Therapie nicht abfallen darf, wird die erfindungsgemäße Beatmungsunterstützung von der Steuereinheit aktiviert. Man erkennt, dass das Volumen langsam angehoben wird. Die erfindungsgemäße Beatmungsunterstützung erhöht den CPAP-Druck für die Inspiration und belässt den CPAP-Druck für die Exspiration (oder senkt den CPAP-Druck für die Exspiration ab). Die erfindungsgemäße Beatmungsunterstützung erhöht den CPAP-Druck für die Inspiration schrittweise, wie aus Fig.2A in der Phase 2 und Phase 3 zu erkennen ist. Dadurch steigen der Fluss und das Volumen wieder an (vergleiche 2B, 2C und 2D).

In der Figur 2D ist das Minuten-Volumen in L pro Minute aufgetragen. Man erkennt, dass das Minuten-Volumen in der Phase 1 absinkt und in der Phase 2 Werte unter 8 L/min einnimmt. Da hier alternativ oder ergänzend zu dem Volumen gemäß Fig. 2C auch ein Minuten-Volumen von 8 L/min als Grenzwert eingestellt sein kann, unter den das Minuten-Volumen bei CPAP-Therapie nicht abfallen darf, wird die erfindungsgemäße Beatmungsunterstützung von der Steuereinheit aktiviert. Man erkennt, dass das Minuten-Volumen in der Phase 3 langsam angehoben wird.

Man erkennt aus dem Verlauf des Maskendruckes Fig. 2A mit dem Fluss der Figur 2B, dass der kurze Druckabfall der spontanen Inspiration 241 des Patienten entspricht. Der Druckabfall korreliert zeitlich mit dem Anstieg des Patientenflusses in Fig. 2B). Dieses Atemsignal des Patienten kann von der Steuerung als Trigger gewertet werden, um den Steuerdruck gezielt für die Inspiration zu erhöhen. Bei Unterschreitung des angestrebten Volumens (Tidalvolumen oder Minutenvolumen) oder bei Unterschreitung der angestrebten Sauerstoffsättigung SpO2 oder bei Überschreiten des angestrebten etCO2-Wertes (endexpiratorischer CO2-Wert im Atemgas), wird gewechselt auf einen IPAP-Druck, der größer ist als der eingestellte CPAP, der CPAP wird zum exspiratorischen Druck (EPAP-Druck).

Die aktuelle Druckdifferenz zum vorherigen Atemzug (CPAP oder sich veränderndes IPAP-Niveau) ergibt sich
a. Aus einer Tabelle, die der Differenz (Soll zu Ist Volumen oder Soll zu Ist SpO2 oder Soll Zu Ist CO2) eine Druckerhöhung zuordnet (abgelegt im Speicher)
b. Aus einem Einstellwert durch einen Nutzer, der der Differenz (Soll zu Ist Volumen oder Soll zu Ist SpO2 oder Soll Zu Ist CO2) eine Druckerhöhung zuordnet
c. Aus einer Kombination aus a) und b)
d. Aus einem (selbstlernenden) Algorithmus mit dem Ziel, die (patho)-physiologische Antwort auf eine Druckerhöhung zu schätzen unter Berücksichtigung aller Eingangsgrößen, Druck, Flow, Volumen, Frequenz, SpO2, etCO2

Bei Überschreitung des angestrebten Volumens (Tidalvolumen oder Minutenvolumen) oder bei Überschreiten der angestrebten Sauerstoffsättigung SpO2 oder bei Unterschreiten des angestrebten etCO2-Wertes (endexpiratorischer CO2-Wert) im Atemgas, wird gewechselt auf einen IPAP-Druck, der kleiner ist als der vorher eingestellte IPAP (der CPAP Wert bleibt der EPAP) oder es wird zurück auf den CPAP-Druck gewechselt.

Die aktuelle Druckdifferenz zum IPAP des vorherigen Atemzugs ergibt sich
a. Aus einer Tabelle, die der Differenz (Soll zu Ist Volumen oder Soll zu Ist SpO2 oder Soll Zu Ist CO2) eine Druckverringerung zuordnet (abgelegt im Speicher)
b. Aus einem Einstellwert durch einen Nutzer, der der Differenz (Soll zu Ist Volumen oder Soll zu Ist SpO2 oder Soll Zu Ist CO2) eine Druckverringerung zuordnet
c. Aus einer Kombination aus a) und b)
d. Aus einem (selbstlernenden) Algorithmus mit dem Ziel, die (patho)-physiologische Antwort auf eine Druckverringerung zu schätzen unter Berücksichtigung aller Eingangsgrößen, Druck, Flow, Volumen, Frequenz, SpO2, etCO2)

## Patentansprüche

1. Beatmungsgerät zur Atemgasversorgung (1) umfassend eine Atemgasquelle (2), eine Steuereinheit (3), einen Speicher (5), eine Drucksensoreinrichtung (7) und/oder eine Flusssensoreinrichtung (8), einen auswechselbaren Atemgasschlauch (11), zumindest einen Anschlussstutzen (221, 222) für den Atemgasschlauch und ein Patienteninterface (4), wobei die Steuereinheit (3) eingerichtet und ausgebildet ist, die Atemgasquelle (2) zur Vorgabe eines im Wesentlichen konstanten CPAP-Drucks, der unabhängig von der Atemphase des Patienten aufrechterhalten wird, anzusteuern und wobei die Steuereinheit (3) eingerichtet und ausgebildet ist, aus den Signalen der Drucksensoreinrichtung (7) und/oder der Flusssensoreinrichtung (8)
• die Atemphase - Inspiration und Exspiration - des Patienten zu ermitteln,
• das aktuelle Atemzugvolumen des Patienten während aufeinanderfolgender Inspirationen und Exspirationen zu ermitteln
• zumindest eine erste, vorgegebene Volumenschwelle für das Atemzugvolumen mit dem aktuellen Atemzugvolumen zu vergleichen
• zu bestimmen, ob das aktuelle Atemzugvolumen die erste Volumenschwelle unterschreitet
• in Reaktion auf eine Unterschreitung der ersten Volumenschwelle,
∘ die Atemgasquelle (2) zur Vorgabe eines zweiten Drucks IPAP für das Atemgas für die Inspiration anzusteuern, wobei der zweite Druck IPAP größer ist als der CPAP-Druck und der zweite Druck IPAP schrittweise so lange erhöht wird, bis die vorgegebene Volumenschwelle für das Atemzugvolumen erreicht ist und/oder dass die Steuereinheit (3) den zweiten Druck IPAP von einer Inspiration zur unmittelbar nachfolgenden Inspiration erhöht
∘ die Atemgasquelle (2) zur Vorgabe des CPAP-Drucks für das Atemgas für die Exspiration anzusteuern,
wobei das Beatmungsgerät zumindest ein Ventil (17) aufweist, welches in dem Atemgasschlauch (11) oder in dem Beatmungsgerät angeordnet ist, **dadurch gekennzeichnet, dass**
die Steuereinheit (3) eingerichtet und ausgebildet ist, den CPAP-Druck auf Druckwerte unter 4 hPa vorzugeben, sodass durch das Ventil (17) eine Auswaschung von CO2 der Ausatemluft auch bei geringen Drücken sicher erfolgt.

2. Beatmungsgerät nach Anspruch 1 **dadurch gekennzeichnet, dass** die Steuereinheit (3) den zweiten Druck IPAP schrittweise wieder absenkt, wenn die vorgegebene Volumenschwelle für das Atemzugvolumen überschritten ist.

3. Beatmungsgerät nach zumindest einem der vorhergehenden Ansprüche **dadurch gekennzeichnet, dass** die Steuereinheit (3) den zweiten Druck IPAP auf das CPAP Druckniveau absenkt, wenn die vorgegebene Volumenschwelle für das Atemzugvolumen in einer vorgegeben Weise überschritten ist und insofern wieder die Atemgasquelle (2) zur Vorgabe eines im Wesentlichen konstanten CPAP-Drucks, der unabhängig von der Atemphase des Patienten aufrechterhalten wird, ansteuert.

4. Beatmungsgerät nach zumindest einem der vorhergehenden Ansprüche, wobei das Ventil (17) abhängig von der Atemphase geöffnet oder geschlossen wird und/oder wobei das Ventil (17) in der Inspiration geschlossen wird und in der Exspiration kontrolliert angesteuert und zeitweise geöffnet wird, um eine Ausatmung zu gewährleisten.

5. Beatmungsgerät nach zumindest einem der vorhergehenden Ansprüche, **dadurch gekennzeichnet, dass** der Atemgasschlauch (11) bei dem Wechsel von einem Modus CPAP (61) zu einem IPAP-Modus (6) an dem Beatmungsgerät (1) verbleibt und das Patienten-Ventil (17) für den IPAP-Modus (6) von der Steuereinheit (3) geschaltet wird.

6. Beatmungsgerät nach zumindest einem der vorhergehenden Ansprüche **dadurch gekennzeichnet, dass** die Patientenatmung von der Steuereinheit (3) aus dem Verlauf eines Flusssignales der Flusssensoreinrichtung (8) identifiziert wird und das Ventil (17) abhängig von dem Flusssignal als Trigger betätigt wird und wobei für das Flusssignal und/oder für ein Drucksignal Grenzwerte hinterlegt sind oder einstellbar sind, wobei die Grenzwerte die Triggersensitivität sind.

7. Beatmungsgerät nach zumindest einem der vorhergehenden Ansprüche **dadurch gekennzeichnet, dass** die Steuereinheit (3) zur Sicherstellung der Aufrechterhaltung des CPAP-Druckniveaus während der Schaltvorgänge des Ventils (17) die Atemgasquelle ansteuert, wobei die Steuereinheit (3) den CPAP-Druck zumindest zeitweise absenkt, wenn die Patientenatmung von der Steuereinheit (3) aus dem Verlauf des Flusssignales der Flusssensoreinrichtung (8) als Exspiration identifiziert wird und/oder
die Steuereinheit (3) den CPAP-Druck zumindest zeitweise anhebt, wenn die Patientenatmung von der Steuereinheit (3) aus dem Verlauf des Flusssignales der Flusssensoreinrichtung (8) als Exspiration identifiziert wird.

8. Beatmungsgerät nach zumindest einem der vorhergehenden Ansprüche **dadurch gekennzeichnet, dass** die Steuereinheit (3) für den Modus CPAP (61) das Ventil (17) in der Inspiration geschlossen hält und in der Exspiration kontrolliert ansteuert und zeitweise öffnet, um eine Ausatmung zu gewährleisten, wobei die Patientenatmung, von der Steuereinheit (3), aus dem Verlauf des Flusssignales der Flusssensoreinrichtung (8) identifiziert und das Ventil (17) abhängig von dem Flusssignal als Trigger betätigt wird, wobei zur Sicherstellung der Aufrechterhaltung des CPAP-Druckniveaus, während der Schaltvorgänge des Ventils (17) die Atemgasquelle angesteuert wird.

9. Beatmungsgerät nach zumindest einem der vorhergehenden Ansprüche **dadurch gekennzeichnet, dass** eine Atemanstrengung des Patienten von der Steuereinheit (3) aus dem Verlauf des Flusssignales (8) oder des Drucksignales identifiziert wird und die Steuereinheit (3) die Atemgasquelle unter Vorgabe eines Atemgasflusses oder Atemgasdrucks ansteuert, wenn aus dem Verlauf des Flusssignales (8) oder des Drucksignales eine Inspirationsbemühung des Patienten identifiziert ist.

10. Beatmungsgerät nach zumindest einem der vorhergehenden Ansprüche **dadurch gekennzeichnet, dass** für die Exspiration das Ventil kurz geöffnet wird, sodass der Druck abgebaut ist, und das Ventil anschließend geschlossen wird.

11. Beatmungsgerät nach zumindest einem der vorhergehenden Ansprüche **dadurch gekennzeichnet, dass** das Beatmungsgerät eine Druckgasquelle (19) und zumindest einen Druckschlauch (251) aufweist, der einen Steuerdruck zu dem Ventil (17) leitet.

12. Beatmungsgerät nach zumindest einem der vorhergehenden Ansprüche **dadurch gekennzeichnet, dass** der Atemgasschlauch (11) ein Einschlauchsystem (111) mit Ventil (17) ist oder der Atemgasschlauch (11) ein Zweischlauchsystem (112) mit Ventil (17) ist.

13. Beatmungsgerät nach zumindest einem der vorhergehenden Ansprüche **dadurch gekennzeichnet, dass** der Atemgasschlauch (11) ein Zweischlauchsystem (112) mit einem zugeordneten Ventil (17) ist, wobei sich das Ventil (17) in einem Beatmungsgerätegehäuse benachbart zu dem Stutzen (222) befindet.

14. Beatmungsgerät nach einem der voranstehenden Ansprüche, **dadurch gekennzeichnet, dass** das Patienten-Ventil (17) aus einer Aufnahme des Gehäuses entnehmbar ausgebildet ist, wobei das Patienten-Ventil (17) eine Membran aufweist, die mit einem Steuerdruck beaufschlagt werden kann, um einen Atemgasfluss über das Ventil zu sperren oder freizugeben, wobei das Ventil eine Dichtmembran aufweist, die mit einem Steuerdruck beaufschlagt wird, der das Ventil öffnet oder schließt, wobei der Steuerdruck von der Atemgasquelle (2) erzeugt wird und über einen Steuerschlauch zum Ventil (17) geleitet wird.

## Claims

1. A respirator for breathing gas supply (1), comprising a breathing gas source (2), a control unit (3), a memory (5), a pressure sensor apparatus (7) and/or a flow sensor apparatus (8), a replaceable breathing gas tube (11), at least one connection nozzle (221, 222) for the breathing gas tube, and a patient interface (4), wherein the control unit (3) is configured and designed to actuate the breathing gas source (2) in order to specify a substantially constant CPAP pressure that is maintained regardless of the breathing phase of the patient and wherein the control unit (3) is configured and designed, based on the signals of the pressure sensor apparatus (7) and/or flow sensor apparatus (8),
• to ascertain the breathing phase - inspiration and expiration - of the patient,
• to ascertain the current tidal volume of the patient during successive inspirations and expirations,
• to compare at least one first, specified volume threshold for the tidal volume with the current tidal volume,
• to determine whether the current tidal volume is lower than the first volume threshold,
• in response to said current tidal volume being lower,
∘ to actuate the breathing gas source (2) in order to specify a second pressure IPAP for the breathing gas for the inspiration, wherein the second pressure IPAP is greater than the CPAP pressure and the second pressure IPAP is increased stepwise until the specified volume threshold for the tidal volume is reached and/or in that the control unit (3) increases the second pressure IPAP from one inspiration to the immediately subsequent inspiration,
∘ to actuate the breathing gas source (2) in order to specify the CPAP pressure for the breathing gas for the expiration,
wherein the respirator comprises at least one valve (7) which is arranged in the breathing gas tube (11) or in the respirator, **characterized in that** the control unit (3) is configured and designed to specify the CPAP pressure to pressure values below 4 hPa, such that CO2 is reliably washed out of the exhalatory air by means of the valve (17) even at low pressures.

2. The respirator according to claim 1, **characterized in that** the control unit (3) reduces the second pressure IPAP stepwise again if the specified volume threshold for the tidal volume is exceeded.

3. The respirator according to at least one of the preceding claims, **characterized in that** the control unit (3) reduces the second pressure IPAP to the CPAP pressure level if the specified volume threshold for the tidal volume is exceeded in a specified manner and thus actuates the breathing gas source (2) again in order to specify a substantially constant CPAP pressure that is maintained regardless of the breathing phase of the patient.

4. The respirator according to at least one of the preceding claims, wherein the valve (17) is opened or closed depending on the breathing phase and/or wherein the valve (17) is closed during inspiration and actuated in a controlled manner and temporarily opened during expiration in order to ensure exhalation.

5. The respirator according to at least one of the preceding claims, **characterized in that** the breathing gas tube (11) remains on the respirator (1) during a change from a mode CPAP (61) to an IPAP mode (6) and the patient valve (17) is switched by the control unit (3) for the IPAP mode (6).

6. The respirator according to at least one of the preceding claims, **characterized in that** the patient's breathing is identified by the control unit (3) from the progression of a flow signal of the flow sensor apparatus (8) and the valve (17) is activated depending on the flow signal as a trigger and wherein limit values are stored or are adjustable for the flow signal and/or for a pressure signal, wherein the limit values are the trigger sensitivity.

7. The respirator according to at least one of the preceding claims, **characterized in that** the control unit (3) actuates the breathing gas source in order to ensure maintenance of the CPAP pressure level during the switching processes of the valve (17), wherein the control unit (3) at least temporarily reduces the CPAP pressure if the patient's breathing is identified as expiration by the control unit (3) from the progression of the flow signal of the flow sensor apparatus (8) and/or
the control unit (3) at least temporarily raises the CPAP pressure if the patient's breathing is identified as expiration by the control unit (3) from the progression of the flow signal of the flow sensor apparatus (8).

8. The respirator according to at least one of the preceding claims, **characterized in that**, for the mode CPAP (61), the control unit (3) keeps the valve (17) shut during inspiration and actuates same in a controlled manner and temporarily opens same during expiration in order to ensure exhalation, wherein the patient's breathing is identified by the control unit (3) from the progression of the flow signal of the flow sensor apparatus (8) and the valve (17) is activated depending on the flow signal as a trigger, wherein the breathing gas source is actuated during the switching processes of the valve (17) in order to ensure maintenance of the CPAP pressure level.

9. The respirator according to at least one of the preceding claims, **characterized in that** a respiratory effort of the patient is identified by the control unit (3) from the progression of the flow signal (8) or pressure signal and the control unit (3) actuates the breathing gas source with the specification of a breathing gas flow or breathing gas pressure if inspiratory effort of the patient is identified from the progression of the flow signal (8) or pressure signal.

10. The respirator according to at least one of the preceding claims, **characterized in that** the valve is opened briefly for expiration, such that the pressure is released, and the valve is then closed.

11. The respirator according to at least one of the preceding claims, **characterized in that** the respirator comprises a compressed gas source (19) and at least one pressure tube (251) which directs a control pressure to the valve (17).

12. The respirator according to at least one of the preceding claims, **characterized in that** the breathing gas tube (11) is a one-tube system (111) with a valve (17) or the breathing gas tube (11) is a two-tube system (112) with a valve (17).

13. The respirator according to at least one of the preceding claims, **characterized in that** the breathing gas tube (11) is a two-tube system (112) with an assigned valve (17), wherein the valve (17) is located in a respirator housing adjacent to the nozzle (222).

14. The respirator according to one of the preceding claims, **characterized in that** the patient valve (17) is designed to be removable from a receiving portion of the housing, wherein the patient valve (17) comprises a membrane to which a control pressure can be applied in order to block or release a breathing gas flow via the valve, wherein the valve comprises a sealing membrane to which a control pressure is applied which opens or closes the valve, wherein the control pressure is generated by the breathing gas source (2) and is directed via a control tube to the valve (17).

## Revendications

1. Ventilateur destiné à l'alimentation en gaz respiratoire (1), comportant une source de gaz respiratoire (2), une unité de commande (3), une mémoire (5), un dispositif de détection de pression (7) et/ou un dispositif de détection de flux (8), un tube de gaz respiratoire (11) remplaçable, au moins une tubulure de raccordement (221, 222) pour le tube de gaz respiratoire et une interface patient (4), dans lequel l'unité de commande (3) est configurée et conçue pour commander la source de gaz respiratoire (2) de manière à déterminer une pression CPAP essentiellement constante, laquelle est maintenue indépendamment de la phase respiratoire du patient, et dans lequel l'unité de commande (3) est configurée et conçue pour, à partir des signaux du dispositif de détection de pression (7) et/ou du dispositif de détection de flux (8)
• détecter la phase respiratoire - inspiration et expiration - du patient,
• détecter le volume respiratoire du patient pendant des inspirations et des expirations consécutives,
• comparer au moins un premier seuil de volume prédéterminé du volume respiratoire avec le volume respiratoire actuel
• définir si le volume respiratoire actuel est inférieur au premier seuil de volume
• en réaction au fait que celui-ci est inférieur au premier seuil de volume,
∘ commander la source de gaz respiratoire (2) de manière à déterminer une deuxième pression IPAP du gaz respiratoire pour l'inspiration, dans lequel la deuxième pression IPAP est supérieure à la pression CPAP et la deuxième pression IPAP est augmentée progressivement jusqu'à ce que le seuil de volume prédéterminé du volume respiratoire soit atteint et/ou l'unité de commande (3) augmente la deuxième pression IPAP entre une inspiration et l'inspiration suivante
∘ commander la source de gaz respiratoire (2) de manière à déterminer la pression CPAP du gaz respiratoire pour l'expiration,
dans lequel le ventilateur présente au moins une valve disposée dans le tube de gaz respiratoire (11) ou dans le ventilateur, **caractérisé en ce que**
l'unité de commande (3) est configurée et conçue pour déterminer la pression CPAP à des valeurs de pression inférieures à 4 hPa, de manière à ce qu'un lessivage de CO2 de l'air expiré s'effectue de manière sûre à travers la valve (17) même à des faibles pressions.

2. Ventilateur selon la revendication 1, **caractérisé en ce que** l'unité de commande (3) abaisse de nouveau progressivement la deuxième pression IPAP une fois que le seuil de volume prédéterminé du volume respiratoire est dépassé.

3. Ventilateur selon l'une au moins des revendications précédentes, **caractérisé en ce que** l'unité de commande (3) abaisse la deuxième pression IPAP au niveau de pression CPAP lorsque le seuil de volume prédéterminé du volume respiratoire est dépassé d'une manière prédéterminée et commande dès lors de nouveau la source de gaz respiratoire (2) de manière à déterminer une pression CPAP essentiellement constante, laquelle est maintenue indépendamment de la phase respiratoire du patient.

4. Ventilateur selon l'une au moins des revendications précédentes, dans lequel la valve (17) est ouverte ou fermée en fonction de la phase respiratoire et/ou dans lequel la valve (17) est fermée dans l'inspiration et commandée de façon contrôlée dans l'expiration, afin de garantir une exhalaison.

5. Ventilateur selon l'une au moins des revendications précédentes, **caractérisé en ce que** le tube de gaz respiratoire (11) reste sur le ventilateur (1) lors d'un passage d'un mode CPAP (61) à un mode IPAP (6) et la valve de patient (17) est commutée par l'unité de commande (3) pour le mode IPAP (6).

6. Ventilateur selon l'une au moins des revendications précédentes, **caractérisé en ce que** la respiration du patient est identifiée par l'unité de commande (3) à partir d'une évolution d'un signal de flux du dispositif de détection de flux (8) et la valve (17) est actionnée en fonction du signal de flux en tant que déclencheur et dans lequel des valeurs limites sont enregistrées ou peuvent être réglées pour le signal de flux et/ou pour un signal de pression, dans lequel les valeurs limites sont la sensibilité de déclenchement.

7. Ventilateur selon l'une au moins des revendications précédentes, **caractérisé en ce que** l'unité de commande (3) commande la source de gaz respiratoire pour assurer le maintien du niveau de pression CPAP pendant les opérations de commutation de la valve (17), dans lequel l'unité de commande (3) abaisse au moins temporairement la pression CPAP lorsque la respiration du patient est identifiée en tant qu'expiration par l'unité de commande (3) à partir de l'évolution du signal de flux du dispositif de détection de flux (8) et/ou
l'unité de commande (3) élève au moins temporairement la pression CPAP lorsque la respiration du patient est identifiée en tant qu'expiration par l'unité de commande (3) à partir du signal de flux du dispositif de détection de flux (8).

8. Ventilateur selon l'une au moins des revendications précédentes, **caractérisé en ce que** l'unité de commande (3) maintient la valve (17) fermée dans l'inspiration pour le mode CPAP (61) et commande celle-ci de façon contrôlée et l'ouvre temporairement dans l'expiration, afin de garantir une exhalaison, dans lequel la respiration du patient est identifiée par l'unité de commande (3) à partir de l'évolution du signal de flux du dispositif de détection de flux (8) et la valve (17) est actionnée en fonction du signal de flux en tant que déclencheur, dans lequel la source de gaz respiratoire est commandée pendant les opérations de commutation de la valve (17) pour assurer le maintien du niveau de pression CPAP.

9. Ventilateur selon l'une au moins des revendications précédentes, **caractérisé en ce que** l'effort respiratoire du patient est identifié par l'unité de commande (3) à partir de l'évolution du signal de flux (8) ou du signal de pression et l'unité de commande (3) commande la source de gaz respiratoire sur la base d'un flux de gaz respiratoire ou d'une pression de gaz respiratoire lorsqu'un effort d'inspiration du patient est identifié à partir de l'évolution du signal de flux (8) ou du signal de pression.

10. Ventilateur selon l'une au moins des revendications précédentes, **caractérisé en ce que** pour l'expiration, la valve est ouverte brièvement, de manière à réduire la pression, et la valve est ensuite fermée.

11. Ventilateur selon l'une au moins des revendications précédentes, **caractérisé en ce que** le ventilateur présente une source de gaz de pression (19) et au moins un tube de pression (251), lequel guide une pression de commande vers la valve (17).

12. Ventilateur selon l'une au moins des revendications précédentes, **caractérisé en ce que** le tube de gaz respiratoire (11) est un système à un tube (111) doté d'une valve (17) ou **en ce que** le tube de gaz respiratoire (11) est un système à deux tubes (112) doté d'une valve (17).

13. Ventilateur selon l'une au moins des revendications précédentes, **caractérisé en ce que** le tube de gaz respiratoire (11) est un système à deux tubes (112) doté d'une valve (17) associée, dans lequel la valve (17) se situe dans un boîtier de ventilateur à proximité de la tubulure (222).

14. Ventilateur selon l'une des revendications précédentes, **caractérisé en ce que** la valve de patient (17) est conçue de manière à pouvoir être retirée d'un logement du boîtier, dans lequel la valve de patient (17) présente une membrane susceptible d'être soumise à une pression de commande, afin de bloquer ou de libérer un flux de gaz respiratoire par le biais de la valve, dans lequel la valve présente une membrane d'étanchéité soumise à une pression de commande permettant d'ouvrir ou de fermer la valve, dans lequel la pression de commande est produite par la source de gaz respiratoire (2) et guidée vers la valve (17) par le biais d'un tube de commande.
